(19) Europäisches Patentamt
European Patent Office
Office européen des brevets

(11) Publication number: **0 293 146**
**A1**

(12) **EUROPEAN PATENT APPLICATION**

(21) Application number: 88304631.0

(22) Date of filing: 23.05.88

(51) Int. Cl.4: A61K 31/435 , A61K 31/47 , C07D 491/048

(30) Priority: 26.05.87 JP 128672/87
26.05.87 JP 128673/87
28.05.87 JP 132946/87

(43) Date of publication of application:
30.11.88 Bulletin 88/48

(84) Designated Contracting States:
AT BE CH DE ES FR GB IT LI NL SE

(71) Applicant: Kissei Pharmaceutical Co Ltd
No. 19-48 Yoshino
Matsumoto-shi Nagano(JP)

(72) Inventor: Kinoshita, Yukihiko
1732-9, Oaza Shimadachi
Matsumoto-shi Nagano(JP)
Inventor: Ikeguchi, Seiichi
5280, Kisofukushima-machi
Kiso-gun Nagano(JP)
Inventor: Tsutsumi,Naoyuki
2-34 Nomizomokko 1-chome
Matsumoto-shi Nagano(JP)
Inventor: Ajisawa, Yukiyoshi
17-1, Minato 4-chome
Okaya-shi Nagano(JP)
Inventor: Ujiie, Arao
6651-31, Takibe Toyoshina-machi
Minamiazumi-gun Nagano(JP)

(74) Representative: Pearce, Anthony Richmond et al
MARKS & CLERK Alpha Tower Suffolk Street
Queensway Birmingham B1 1TT(GB)

(54) **Benzofuro [3,2-c] quinoline compounds.**

(57) Benzofuro[3,2-c]quinoline compounds of the general formula:

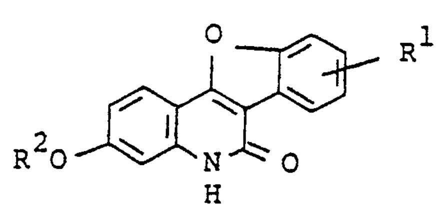

wherein $R^1$ represents a hydrogen atom, a hydroxy group or an unsubstituted or substituted straight- or branched-chain alkoxy group having 1 to 10 carbon atoms wherein the substituent is selected from a hydroxy group, a carboxy group, a carbamoyl group and an alkoxycarbonyl group having 2 to 7 carbon atoms; $R^2$ represents a hydrogen atom or an unsubstituted or substituted straight- or branched-chain alkyl group having 1 to 10 carbon atoms wherein the substituent is selected from a hydroxy group, a carboxy

EP 0 293 146 A1

group, a carbamoyl group and an alkoxycarbonyl group having 2 to 7 carbon atoms, and the pharmaceutically acceptable salts thereof, possess a strong inhibitory action on bone resorption and a stimulatory effect on ossification and are thus useful for the prevention or treatment of osteoporosis.

# BENZOFURO[3,2-c]QUINOLINE COMPOUNDS

## FIELD OF THE INVENTION

The present invention relates to benzofuro[3,2-c]quinoline compounds which are useful as a therapeutic agent. More particularly, the present invention relates to a pharmaceutical composition containing, as an active ingredient, a benzofuro[3,2-c]quinoline compound or a pharmaceutically acceptable salt thereof, represented by the general formula:

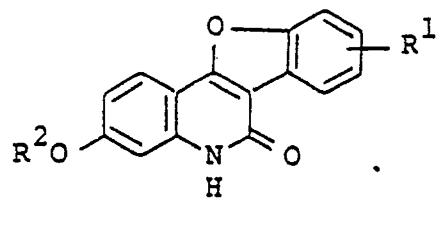

wherein $R^1$ represents a hydrogen atom, a hydroxy group or an unsubstituted or substituted straight- or branched-chain alkoxy group having 1 to 10 carbon atoms wherein the substituent is selected from a hydroxy group, a carboxy group, a carbamoyl group and an alkoxycarbonyl group having 2 to 7 carbon atoms; $R^2$ represents a hydrogen atom or an unsubstituted or substituted straight- or branched-chain alkyl group having 1 to 10 carbon atoms wherein the substituent is selected from a hydroxy group, a carboxy group, a carbamoyl group and an alkoxycarbonyl group having 2 to 7 carbon atoms; and to a method for the prevention and treatment of osteoporosis by administering the pharmaceutical composition containing the above compound or the salt thereof.

The present invention also relates to novel benzofuro[3,2-c]quinoline compounds or pharmaceutically acceptable salts thereof, represented by the general formula:

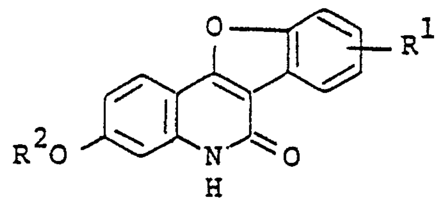

wherein $R^1$ represents a hydrogen atom, a hydroxy group or an unsubstituted or substituted straight- or branched-chain alkoxy group having 1 to 10 carbon atoms wherein the substituent is selected from a hydroxy group, a carboxy group, a carbamoyl group and an alkoxycarbonyl group having 2 to 7 carbon atoms; $R^2$ represents a hydrogen atom or an unsubstituted or substituted straight- or branched-chain alkyl group having 1 to 10 carbon atoms wherein the substituent is selected from a hydroxy group, a carboxy group, a carbamoyl group and an alkoxycarbonyl group having 2 to 7 carbon atoms, with the proviso that, when $R^1$ represents a hydroxy group, $R^2$ can not be a hydrogen atom, and with the further proviso that, when $R^1$ represents a methoxy group, $R^2$ can not be a methyl group.

## BACKGROUND OF THE INVENTION

Osteoporosis is a diseased condition or illness wherein the quantitative loss of bones has progressed beyond a certain limit with no substantial change in chemical composition of the bones. A decrease in the amount of protein, calcium and phosphorus in bones is its physiological feature. Osteoporosis is increased with aging, and is observed most commonly in the elder. The disease usually invades the vertebra, and induces dorsal lumbago and shortening the height of patients. Especially, in the advanced case, the disease

invades the long bone. Therefore, fracture often occurs in a patient suffering from osteoporosis. It is thought that the femur fracture observed in old women and men is almost caused by osteoporosis. The pathogenic factors are varied, including endocrine disorder and nutritional disorder. Therapeutic agents such as vitamine $D_3$, calcium preparations, calcitonin, and phosphorus preparations are employed in the prevention or treatment of osteoporosis, but these are limited in effect to a given subject and can hardly be expected to show a definite effect. Therefore, it has long been desired to develop a pharmaceutical agent having a significant effect.

Recently, it has been reported that a certain compound of 3-phenyl-4H-1-benzopyran-4-ones which is different from the above agents is useful as a therapeutic agent for the prevention or treatment of osteoporosis in Japanese Patent Publication No. 13391/79 and Japanese Patent Application (OPI) Nos. 48924/85, 54379/85, 132917/85, 132976/85. (The term "OPI" as used herein refers to an unexamined Japanese patent application)

Up to now, with regard to benzofuro[3,2-c]quinoline compounds related to those of the present invention, the compounds represented by the following formulae (A), (B) and (C) have been disclosed in Bulletin of the Chemical Society of Japan, Vol. 53, pages 1057-1060 (1980), Journal of Heterocyclic Chemistry, Vol. 16, pages 487-491 (1979), and ibidem, Vol. 21, pages 737-739 (1984).

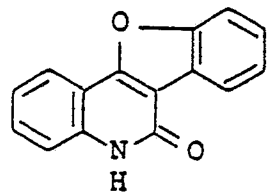

(A)

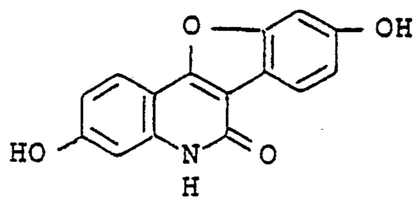

(B)

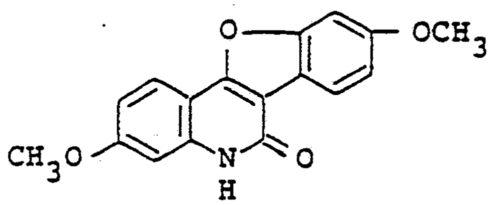

(C)

These compounds were prepared in order to investigate their chemical reactivities and to test their activities as mutagens, carcinogens, and anti-tumor substances, but there is no specific disclosure as to their pharmacological activities in these references. Furthermore, it has not been reported in any literature references that any of benzofuro[3,2-c]quinoline compounds is useful for the prevention or treatment of osteoporosis, and thus, the pharma ceutical composition according to the present invention include the use of the above known compounds (A), (B) and (C).

The present inventors studied more effective drugs for the prevention or treatment of osteoporosis. As a result, the inventors have found that a certain compound of benzofuro[3,2-c]quinoline compounds or a pharmaceutically acceptable salt thereof exhibits a strong inhibitory action on bone resorption and a stimulatory effect on ossification, and thus that they are useful as a therapeutic agent for the prevention and treatment of osteoporosis.

## SUMMARY OF THE INVENTION

An object of the present invention is to provide novel benzofuro[3,2-c]quinoline compounds which exhibit a strong inhibitory action on bone resorption and a stimulatory effect on ossification.

Another object of the present invention is to provide pharmaceutical compositions comprising a benzofuro[3,2-c]quinoline compound or pharmaceutically acceptable salt thereof.

A further object of the present invention is to provide methods for the prevention or treatment of osteoporosis by administering a benzofuro[3,2-c]quinoline compound or pharmaceutically acceptable salt thereof.

Other objects, features and advantages of the present invention will be apparent from the following description of the invention.

## DETAILED DESCRIPTION OF THE INVENTION

The benzofuro[3,2-c]quinoline compounds of the present invention and pharmaceutically acceptable salts thereof exhibit an inhibitory action on bone resorption and a stimulatory effect on ossification, and thus are useful as a therapeutic agent for the prevention or treatment of osteoporosis.

The benzofuro[3,2-c]quinoline compounds of the present invention can be prepared according to methods known per se. That is, of the benzofuro[3,2-c]quinoline compounds of the present invention represented by the general formula (I);

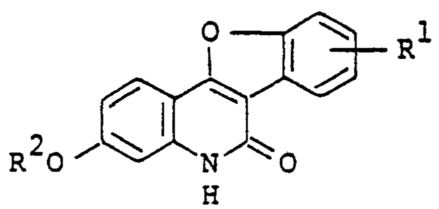

(I)

wherein $R^1$ and $R^2$ have the same meanings as defined above; the compounds represented by the general formula (Ia):

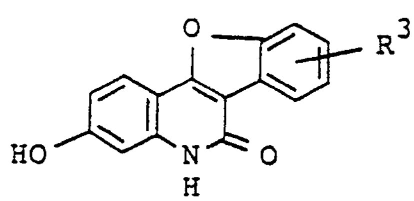

(Ia)

wherein $R^3$ represents a hydrogen atom or a hydroxy group, can be prepared by reacting m-anisidine with diethyl mono- or dialkoxyphenylmalonate in an inert organic solvent to obtain 4-hydroxy-7-methoxy-3-(mono- or dialkoxyphenyl)-2-quinolone, and then treating the obtained compound with pyridine hydrochloride.

Of the benzofuro[3,2-c]quinoline compounds of the general formula (I) of the present invention, the compounds represented by the general formulae (Ib) and (Ib′):

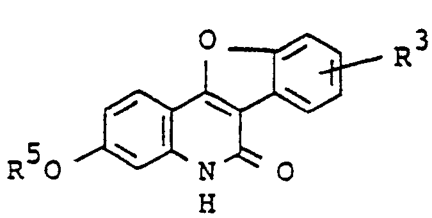

(Ib)

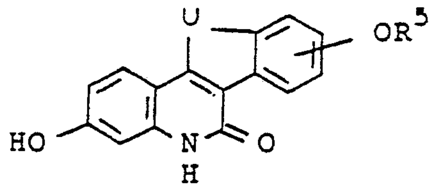

(Ib')

wherein $R^5$ represents an unsubstituted or substituted straight- or branched-chain alkyl group having 1 to 10 carbon atoms wherein the substituent is selected from a hydroxy group, a carboxy group, a carbamoyl group and an alkoxycarbonyl group having 2 to 7 carbon atoms, $R^3$ has the same meaning as defined above; can be prepared by reacting a compound represented by the general formula (Ia) with a compound represented by the following general formula (III) in an equimolar amount to the compound of the general formula (Ia):

$R^5$ - X (III)

wherein X represents an acid residual group, $R^5$ has the same meaning as defined above, in the presence of a basic substance such as sodium hydride in an inert organic solvent such as N,N-dimethylformamide.

Furthermore, of the benzofuro[3,2-c]quinoline compounds of the general formula (I) of the present invention, the compounds represented by the general formula (Ic):

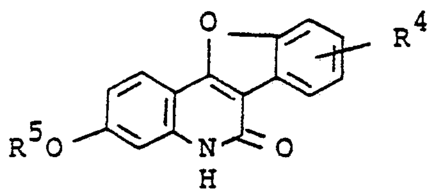

(Ic)

wherein $R^4$ represents hydrogen atom or an unsubstituted or substituted alkoxy group having 1 to 10 carbon atoms wherein the substituent is selected from a hydroxy group, a carboxy group, a carbamoyl group and an alkoxycarbonyl group having 2 to 7 carbon atoms, $R^5$ has the same meaning as defined above; can be prepared by reacting a compound of the general formula (Ia) with a compound of the general formula (III) in one or more molar amounts to the compound of the general formula (Ia) in the presence of a basic substance such as sodium hydride in an inert organic solvent such as N,N-dimethylformamide.

The process for the production of the compound (Ia) can be preferably carried out by heating m-anisidine with diethyl mono- or dialkoxyphenylmalonate in an equimolar amount under reflux for about 1 to 3 hours in diphenyl ether to obtain 4-hydroxy-7-methoxy-3-(mono- or dialkoxyphenyl)-2-quinolone, and heating the obtained compound in pyridine hydrochloride under reflux for about 1 to 3 hours.

The processes for the production of the compounds (Ib), (Ib') and (Ic) can also preferably carried by O-alkylating a compound of the general formula (Ia) with a compound of the general formula (III) according usual manners described in literature.

The desired product can be isolated from the reaction mixture and purified by conventional manner such as extraction, recrystallization, and chromatography.

Of the benzofuro[3,2-c]quinoline compounds of the general formula (I) of the present invention, the compounds where $R^1$ and $R^2O$ groups are different alkoxy group each other can be prepared by O-alkylation from a compound where one of $R^1$ and $R^2O$ group is an alkoxy group and other is a hydroxy group. As diethyl mono- or dialkoxyphenylmalonates used as starting materials in the present invention, diethyl 2-methoxyphenylmalonate, diethyl 2,3-, 2,4- or 2,5-dimethylphenylmalonate can be employed, and they can be prepared by the method disclosed in Journal of Heterocyclic Chemistry, Vol. 21, pages 737-739, (1984).

The compounds represented by the general formula (III) used as another strating material in the present invention are commercially available.

Of the benzofuro[3,2-c]quinoline compound represented by the general formula (I) of the present invention, compounds where a hydroxy group is placed on 8 or 9-position is preferred. The most preferred compounds are 3,8-dihydroxy-5H-benzofuro[3,2-c]quinolin-6-one, 3,9-dihydroxy-5H-benzofuro[3,2-c]quinolin-6-one, 9-hydroxy-3-isoproxy-5H-benzofuro[3,2-c]quinolin-6-one and 9-hydroxy-3-(3-hydroxypropoxy)-5H-benzofuro[3,2-c]quinolin-6-one.

The benzofuro[3,2-c]quinoline compounds represented by the general formula (I) having a phenolic hydroxy group can be converted according to conventional methods into pharmaceutically acceptable salts thereof. Examples of such pharmaceutically acceptable salts include pharmaceutically acceptable inorganic basic salts such as a sodium salt and calcium salt.

The benzofuro[3,2-c]quinoline compounds represented by the general formula (I) of the present invention and pharmaceutically acceptable salts thereof possess a strong inhibitory action on bone resorption and a stimulatory effect on ossification, for example, the benzofuro[3,2-c]quinoline compounds produce a desired effect at a $10^{-4}$ - $10^{-5}$ molar concentration when determined by the in vitro experiment using femur of chick embryo.

The benzofuro[3,2-c]quinoline compounds of the general formula (I) of the present invention and the pharmaceutically acceptable salts thereof can be administered in various dosage forms depending upon the intended therapy. Typical dosage forms which can be used are tablets, pills, powders, suspensions, emulsions, granules, capsules, suppositories, and injectable preparations.

In molding the pharmaceutical compositions into a tablet form, a wide variety of conventional carriers known in the art can be used. Examples of suitable carriers are excipients such as glucose, lactose, starch, cacao butter, hardened vegetable oils, kaolin and talc, binders such as gum arabic powder, tragacanth powder, and ethanol, and disintegrators such as laminaria and agar. The tablets, if desired, can be coated into sugar-coated tablets, gelatin-coated tablets, film-coated tablets, or tablets coated with two or more layers.

When the pharmaceutical composition is formulated into an injectable preparation, the resulting solution and suspension are preferably sterilized, and are isotonic with respect to blood. In making the pharmaceutical composition into a solution or suspension, all diluents customarily used in the art can be employed. Examples of suitable diluents are water, ethyl alcohol, propylene glycol, ethoxylate isostearyl alcohol, polyoxyethylene sorbitol, and sorbitan esters. Sodium chloride, glucose or glycerol can be incorporated into a therapeutic agent in an amount sufficient to prepare an isotonic solution. The therapéutic agent may further contain ordinary dissolving aids, buffers, pain-alleviating agents, and optionally, coloring agents, fragrances, flavors, sweeteners, and other pharmacologically active agents which are known in the art.

The dosage of the benzofuro[3,2-c]quinoline compounds of the present invention and pharmaceutically acceptable salts thereof can be in the range from about 10 mg to 1,000 mg per adult human by oral administration per day, or from about 1 mg to 100 mg per adult human by parenteral administration per day in multiple doses depending upon the type of disease, the severity of condition to be treated, and the like.

The present invention is further illustrated in more detail by way of the following Examples. The melting points of the products obtained were uncorrected.

Example 1

3-Hydroxy-5H-benzofuro[3,2-c]quinolin-6-one (compound 1)

A mixture of 2.7 g of m-anisidine and 5.32 g of diethyl 2-methoxyphenylmalonate in 20 ml of diphenyl ether was placed in a flask equipped with an air condenser, and heated for 2.5 hours at 270°-290°C for 2.5 hours. After cooling, to the reaction mixture was added 80 ml of diethyl ether. The precipitates were collected by filtration, and washed with diethyl ether to obtain 5.39 g of 4-hydroxy-7-methoxy-3-(2-methoxyphenyl)-2-quinolone (yield: 90.7%).

melting point: >300°C
IR (KBr): $\nu$co 1620 $cm^{-1}$
NMR ($d_6$-DMSO)
$\delta$: 3.69(s, 3H), 3.81(s, 3H), 6.75-6.79(m, 2H), 6.93-7.13(m, 3H), 7.29-7.35(m, 1H), 7.76-7.80(m, 1H), 9.56(br-s, 1H), 11.18(s, 1H)
elemental analysis as $C_{17}H_{15}NO_4$

| | C% | H% | N% |
|---|---|---|---|
| Calcd. | 68.68 | 5.09 | 4.71 |
| Found | 68.79 | 5.08 | 4.72 |

A mixture of 5 g of 4-hydroxy-7-methoxy-3-(2- methoxyphenyl)-2-quinolone and 50 g of pyridine hydrochloride was heated under reflux for 2.5-3 hours at 220-250 °C, and the hot reaction mixture was poured into 200-300 g of a rubble-ice. The precipitates were collected by filtration, washed with water, and recrystallized from an alcohol to obtain 1.9 g of 3-hydroxy-5H-benzofuro[3,2-c]quinolin-6-one.

melting point: >300 °C
IR (KBr): $\nu$co 1640 $cm^{-1}$
NMR ($d_6$-DMSO)
$\delta$: 6.92-7.04(m, 2H), 7.52-7.60(m, 2H), 7.89-8.17(m, 3H), 10.49(s, 1H), 11.88(s, 1H)
elemental analysis as $C_{15}H_9NO_3$

| | C% | H% | N% |
|---|---|---|---|
| Calcd. | 71.71 | 3.61 | 5.58 |
| Found | 71.37 | 3.61 | 5.44 |

Example 2

The following compounds were prepared in a similar manner to that described in Example 1 except that diethyl 2,4-dimethoxyphenylmalonate or diethyl 2,5-dimethoxyphenylmalonate was used in place of the diethyl 2-methoxyphenylmalonate.

<u>3,9-Dihydroxy-5H-benzofuro[3,2-c]quinolin-6-one</u> <u>(Compound</u> <u>2)</u>

melting point: >300 °C
IR (KBr): $\nu$co 1640 $cm^{-1}$
NMR ($d_6$-DMSO)
$\delta$: 6.77-6.91(m, 3H), 7.11-7.12(m, 1H), 7.78-8.30(m, 2H), 9.83(s, 1H), 10.22(s, 1H), 11.67(s, 1H)
elemental analysis as $C_{15}H_9NO_4$

| | C% | H% | N% |
|---|---|---|---|
| Calcd. | 67.42 | 3.39 | 5.24 |
| Found | 67.24 | 3.41 | 5.35 |

<u>3,8-Dihydroxy-5H-benzofuro[3,2-c]quinolin-6-one</u> <u>(Compound</u> <u>3)</u>

melting point: >300 °C
IR (KBr): $\nu$co 1660, 1630 $cm^{-1}$
NMR ($d_6$-DMSO)
$\delta$: 6.77-6.90(m, 3H), 7.39-7.85(m, 3H), 9.46(s, 1H), 10.31(s, 1H), 11.64(s, 1H)
elemental analysis as $C_{15}H_9NO_4$

| | C% | H% | N% |
|---|---|---|---|
| Calcd. | 67.42 | 3.39 | 5.24 |
| Found | 67.71 | 3.45 | 5.54 |

Example 3

3-Isopropoxy-5H-benzofuro[3,2-c]quinolin-6-one (Compound 4

To a solution of 500 mg of 3-hydroxy-5H-benzofuro[3,2-c]quinolin-6-one in 20 ml of N,N-dimethylformamide was added 100 mg of a 60% sodium hydride (dispersion in an oil), and then the mixture was stirred for a little while. To the mixture was added 0.5 ml of isopropyl iodide under ice-cooling, and then the mixture was stirred overnight at room temperature. The reaction mixture was evaporated under reduced pressure, and to the residue was washed successively with a 5% aqueous sodium hydroxide solution, water and diethyl ether, and recrystallized from ethyl acetate to obtain 214 mg of 3-isopropoxy-5H-benzofuro[3,2-c]quinolin-6-one.

melting point: 278-280 °C
IR (KBr): $\nu$co 1640 $cm^{-1}$
NMR ($CDCl_3$)
$\delta$: 1.34(d, 6H), 4.69(quint. 1H), 6.94-7.03(m, 2H), 7.42-7.51(m, 2H), 7.99-8.07(m, 3H), 11.79(s, 1H)
elemental analysis as $C_{18}H_{15}NO_3$

| | C% | H% | N% |
|---|---|---|---|
| Calcd. | 73.71 | 5.15 | 4.78 |
| Found | 73.56 | 5.15 | 4.67 |

Example 4

The following compounds were prepared in a similar manner to that described in Example 3 except that ethyl 3-chloro-1-propanol or ethyl-$\alpha$-bromopropionate in place of isopropyl iodide used in Example 3.

3-(3-Hydroxypropoxy)-5H-benzofuro[3,2-c]quinolin-6-one (Compound 5)

melting point: 275-280 °C
IR (KBr): $\nu$co 1640 $cm^{-1}$
NMR ($d_6$-DMSO)
$\delta$: 2.04(t, 2H), 3.71(q, 2H), 4.26(t, 2H), 7.08-7.15(m, 2H), 7.51-7.63(m, 2H), 7.92-8.20(m, 3H), 11.96(s, 1H)
elemental analysis as $C_{18}H_{15}NO_4$

| | C% | H% | N% |
|---|---|---|---|
| Calcd. | 69.89 | 4.89 | 4.53 |
| Found | 69.61 | 4.85 | 4.43 |

3-(1-Ethoxycarbonylethoxy)-5H-benzofuro[3,2-c]quinolin-6-one (Compound 6)

melting point: 209-212 °C
IR (KBr): νco 1730, 1655 $cm^{-1}$
NMR ($d_6$-DMSO)
δ: 1.33(t, 3H), 1.69(d, 3H), 4.30(q, 2H), 5.15(q, 1H), 7.06-7.10(m, 2H), 7.57-7.62(m, 2H), 7.95-8.20(m, 3H), 12.02(s, 1H)
elemental analysis as $C_{20}H_{17}NO_5$

| | C% | H% | N% |
|---|---|---|---|
| Calcd. | 68.37 | 4.88 | 3.99 |
| Found | 68.58 | 4.83 | 4.12 |

Example 5

3-(1-Carboxyethoxy)-5H-benzofuro[3,2-c]quinolin-6-one (Compound 7)

To a solution of 200 mg of 3-(1-ethoxycarbonylethoxy)-5H-benzofuro[3,2-c]quinolin-6-one in 10 ml of ethanol was added 20 ml of a 10% aqueous sodium hydroxide solution, and then the mixture was heated on a water bath for 1 hour at 80 °C. After cooling, the reaction mixture was acidified by adding hydrochloric acid. The precipitates were collected by filtration, washed with water, and recrystallized from an aqueous alcohol solution to obtain 190 mg of 3-(1-carboxy ethoxy)-5H-benzofuro[3,2-c]quinolin-6-one.

melting point: 260-266 °C
IR (KBr): νco 1750, 1730, 1640 $cm^{-1}$
NMR ($d_6$-DMSO)
δ: 1.69(d, 3H), 5.05(q, 1H), 7.04-7.08(m, 2H), 7.54-7.64(m, 2H), 7.90-7.97(m, 1H), 8.09-8.23(m, 2H), 11.99(s, 1H), 13.30(br-s, 1H)
elemental analysis as $C_{18}H_{13}NO_5$

| | C% | H% | N% |
|---|---|---|---|
| Calcd. | 66.87 | 4.05 | 4.33 |
| Found | 66.55 | 4.05 | 4.55 |

Example 6

3,9-Diisopropoxy-5H-benzofuro[3,2-c]quinolin-6-one (Compound 8)

9-Hydroxy-3-isopropoxy-5H-benzofuro[3,2-c]quinolin-6-one (Compound 9)

To a solution of 267 mg of 3,g-dihydroxy-5H-benzofuro[3,2-c]quinolin-6-one in 20 ml of N,N-dimethylformamide was added 80 mg of a 60% sodium hydride, and the mixture was stirred for some time. To the mixture was added 0.4 ml of isopropyl iodide, and the mixture was stirred overnight at room temperature. The reaction mixture was evaporated under reduced pressure, and the residue was dissolved in chloroform. The solution was extracted with a 5% aqueous sodium hydroxide solution. The organic layer was washed with water, dried over anhydrous magnesium sulfate, and evaporated under reduced pressure. The residue was recrystallized from a mixture of ethyl acetate and n-hexane to obtain 130 mg of 3,9-diisopropoxy-5H-benzofuro[3,2-c]quinolin-6-one.

The aqueous layer was acidified by adding hydrochloric acid, and the precipitates were purified by silica gel column chromatography (eluent: dichloromethane/ether/methanol = 20/20/1) to obtain 45 mg of 3-isopropoxy-g-hydroxy-5H-benzofuro[3,2-c]quinolin-6-one.

3,9-Diisopropoxy-5H-benzofuro[3,2-c]quinolin-6-one (Compound 8)

melting point: 246.5-250 °C
IR (KBr): $\nu$co 1660 $cm^{-1}$
NMR ($d_6$-DMSO)
$\delta$: 1.43-1.47(m, 12H), 4.73-4.90(m, 2H), 7.04-7.16(m, 3H), 7.54-7.55(m, 1H), 7.98-8.03(m, 2H), 11.85(s, 1H)
elemental analysis as $C_{21}H_{21}NO_4$

| | C% | H% | N% |
|---|---|---|---|
| Calcd. | 71.78 | 6.02 | 3.99 |
| Found | 71.52 | 6.01 | 4.15 |

9-Hydroxy-3-isopropoxy-5H-benzofuro[3,2-c]quinolin-6-one (Compound 9)

melting point: 260-265 °C
IR (KBr): $\nu$co 1660 $cm^{-1}$
NMR ($d_6$-DMSO)
$\delta$: 1.45(d, 6H), 4.79(quint, 1H), 7.01-7.26(m, 4H), 7.91-8.03(m, 2H), 10.01(s, 1H), 11.82(s, 1H)
elemental analysis as $C_{18}H_{15}NO_4$

| | C% | H% | N% |
|---|---|---|---|
| Calcd. | 69.89 | 4.89 | 4.53 |
| Found | 69.62 | 4.83 | 4.41 |

Example 7

The following compounds were prepared in a similar manner to that described in Example 4 except that 3-chloro-1-propanol or ethyl $\alpha$-bromopropionate were used in place of isopropyl iodide used in Example 4.

3,9-Bis(3-hydroxypropoxy)-5H-benzofuro[3,2-c]quinolin-6-one (Compound 10)

melting point: 225-232 °C
IR (KBr): νco 1660, 1630 $cm^{-1}$
NMR ($d_6$-DMSO)
δ: 1.99-2.08(m, 4H), 3.67-7.45(m, 4H), 4.24(t, 2H), 4.265(t, 2H), 7.05-7.19(m, 3H), 7.54(d, 1H), 7.91-8.04(m, 2H), 11.91(s, 1H)
elemental analysis as $C_{21}H_{21}NO_6$

| | C% | H% | N% |
|---|---|---|---|
| Calcd. | 65.78 | 5.52 | 3.65 |
| Found | 65.53 | 5.41 | 3.59 |

9-Hydroxy-3-(3-hydroxypropoxy)-5H-benzofuro[3,2-c]quinolin-6-one (Compound 11)

melting point: 263.5-267 °C
IR (KBr): νco 1640 $cm^{-1}$
NMR ($d_6$-DMSO)
δ: 2.03(t, 2H), 3.71(q, 2H), 4.26(t, 2H), 7.06-7.26(m, 4H), 7.91-8.04(m, 2H), 10.01(s, 1H), 11.90(s, 1H)
elemental analysis as $C_{18}H_{15}NO_5$

| | C% | H% | N% |
|---|---|---|---|
| Calcd. | 66.45 | 4.65 | 4.31 |
| Found | 66.16 | 4.53 | 4.29 |

3,9-Bis(1-ethoxycarbonylethoxy)-5H-benzofuro[3,2-c]quinolin-6-one (Compound 12)

melting point: 187-194 °C
IR (KBr): νco 1750, 1670, 1630 $cm^{-1}$
NMR ($d_6$-DMSO)
δ: 1.30(t, 3H), 1.33(t, 3H), 1.68(d, 6H), 4.24-4.34(m, 4H), 5.14(q, 1H), 5.25(q, 1H), 7.05-7.51(m, 1H), 8.01-8.07(m, 2H), 11.98(s, 1H)
elemental analysis as $C_{25}H_{25}NO_8$

| | C% | H% | N% |
|---|---|---|---|
| Calcd. | 64.23 | 5.39 | 3.00 |
| Found | 63.96 | 5.36 | 2.97 |

3,9-Dimethoxy-5H-benzofuro[3,2-c]quinolin-6-one (Compound 13)

melting point: 299-302 °C
IR (KBr): $\nu$co 1675 $cm^{-1}$
NMR ($d_6$-DMSO)
$\delta$: 3.90(s, 6H), 7.10-7.30(m, 3H), 7.50(d, 1H), 8.10(d, 2H)
elemental analysis as $C_{17}H_{13}NO_4$

| | C% | H% | N% |
|---|---|---|---|
| Calcd. | 69.14 | 4.44 | 4.74 |
| Found | 68.90 | 4.48 | 4.95 |

Example 8

Inhibitory effect on bone resorption

Inhibitory effect of various compounds on bone resorption was tested according to the method described in "Advanced Tissue Cultures For In Vitro Assay And Production" (pages 111-114, edited by Isao Yamane & Hiroyoshi Endo, Published by SOFT SCIENCE, Inc. Tokyo).

Femur was isolated from 10- to 11-day chick embryo. After cleaned from adherent soft tissues, the preparation was precultivated in 1 ml of BGJb-HW2 culture medium containing benzofuro[3,2-c]quinoline derivatives of the present invention at 37 °C for 24 hours using the roller-tube method. Tested compounds were dissolved in dimethylsulfoxide at a concentration of 0.1 M and the solution was diluted with the culture medium to make a final concentration of $10^{-4}$ M of those compounds. In the case of control group, the same volume of dimethylsulfoxide was added.

On the next day, the precultivated femur was further cultivated for 2 hours at 37 °C in 1 ml of fresh culture medium containing 1 $\mu$Ci/ml of $^{45}CaCl_2$ to label the bone mineral with $^{45}$Ca. Then, the cultivated bone was rinsed with a phosphate-buffered saline warmed at 37 °C to remove $^{45}$Ca adhered to the bone. $^{45}$Ca-Labelled bone was again cultivated using the roller-tube method (10 revolutions/hr), and the radioactivity of $^{45}$Ca in an aliquot of the culture medium was determined by liquid scintillation counter at a point of 12, 24, 48 and 72 hours, respectively. The culture medium was freshed at each determination of the radioactivity. After completion of the cultivation, the bone was immersed in 1 ml of 1N HCl for 24 hours to elute all calcium in the bone, and the remaining radioactivity in the bone was determined.

From the obtained data, the rate of remaining radioactivity of the bone to the initial radioactivity of the bone was measured at each observation. Then the eluting rate of bone mineral by osteoclasts was obtained by linear-regression of decay curve for the rate of remaining radioactivity in the bone after 24 to 72 hours of the cultivation, and turnover rate of calcium in the bone mineral accumulated in the cultivated bone was estimated as biological half-life ($T_{1/2}$).

In the case that $T_{1/2}$ of the test compound group of the present invention preparations is larger than that of control group, it shows that these compounds have inhibitory effect on bone resorption. The potency of the inhibition of presented compounds was calculated by the following equation using $T_{1/2}$.

$$\text{Inhibitory potency on bone resorption} = \frac{T_{1/2} \text{ of the test compound group}}{T_{1/2} \text{ of the control group}}$$

The results are shown in the following table as the mean value of 5 observations.

| [Compounds] | [Inhibitory potency on bone resorption] |
|---|---|
| 1 | 1.24 |
| 2 | 1.97 |
| 3 | 2.52 |
| 4 | 1.16 |
| 5 | 1.26 |
| 6 | 1.10 |
| 7 | 1.10 |
| 8 | 1.24 |
| 9 | 2.89 |
| 10 | 1.40 |
| 11 | 2.89 |
| 12 | 1.16 |
| 13 | 1.04 |

Example 9

Stimulatory effect on ossification

Stimulatory effect of various compounds on ossification was tested according to the method described in "Advanced Tissue Cultures For In Vitro Assay And Production" (pages 111-114, edited by Isao Yamane & Hiroyoshi Endo, Published by SOFT SCIENCE, Inc. Tokyo).

Femur was isolated from 9-day chick embryo. After cleaned from adherent soft tissues, one femur of a paired femur was used for a test of compound of the present invention, and the other femur was used as control group. One preparation was placed directly on the inner surface of a glass roller-tube, and 2 ml of BGJb-HW2 culture medium was added to each tube. Each preparation was cultivated at 37° C by the roller-tube method (10 revolutions/hr) for 6 days. During the cultivation, femur length was measured, and the culture medium was freshed every other day. Tested compounds were dissolved in dimethylsulfoxide at a concentration of 0.01 M and the solution was diluted by the culture medium to make a final concentraiton of $10^{-5}$ M of those compounds. In the case of control group, the same volume of dimethylsulfoxide was added.

After completion of the cultivation, the bone was immersed in 1N HCl for 24 hours to elute all calcium in the bone, and the eluted calcium was determined by chelating method using orthocresolphthalein.

The potency of the stimulatory effect on ossification of presented compounds was calculated by the following equation.

$$\text{Stimulatory potency on ossification} = \frac{\text{Amount of calcium in the test compound group}}{\text{Amount of calcium in the control group}}$$

The results are shown in the following table as the mean value of 6 observations.

| [Compounds] | [Stimulatory potency on ossification] |
|---|---|
| 2 | 1.11 |
| 9 | 1.08 |
| 11 | 1.14 |

Example 10

Effect on calcium and phosphorus contents in femur of rats loaded low calcium diets

Twenty male Wistar rats of 3 weeks age were divided into 2 groups. All animals were raised with low calcium diets. Test compound, 3,9-dihydroxy-5H-benzofuro [3,2-c]quinolin-6-one (compound 2) suspended in 0.5% sodium carboxymethyl cellulose solution (CMC) was orally administered to 1 group at a dose of 300 mg/kg once a day for 2 weeks. Another group recieved the same volume of CMC. After completion of the experiment, calcium and phosphorus contents in the femur were determined.

The results were shown in the following table as mean±standard error of the mean of 10 observations.

| | Amount of calcium (mg/femur) | Amount of phosphorus (mg/femur) |
|---|---|---|
| Control group | 4.2±0.62 | 3.2±0.35 |
| Test compound group | 8.1±0.50 | 5.1±0.26 |

Example 11

Acute toxicity, test

3,9-Dihydroxy-5H-benzofuro[3,2-c]quinolin-6-one suspended in 0.5% CMC was administered orally at doses of 1000, 2000 and 3000 mg/kg to 10 male ICR mice of 7 weeks age. No death of animals and no induction of toxic symptom were observed during 7 days after the administration.

Example 12

Tablets

100 Grams of 3,9-dihydroxy-5H-benzofuro[3,2-c]quinolin-6-one were admixed with 95 g of latose and 40 g of Indian corn starch and with 700 ml of a 5% aqueous solution of hydroxypropylcellulose, and then dried. The dried mixture was admixed with 8 g of calcium carboxymethylcellulose and 7 g of calcium stearate and the mixture was shaped into 1000 tablets.

Example 13

Capsules

100 Grams of 3,9-dihydroxy-5H-benzofuro[3,2-c]quinolin-6-one were admixed with 59 g of lactose and 35 g of Indian corn starch. The mixture was admixed with 6 g of talc, and the mixture was charged equally into 1000 hard capsules.

## Claims

1. A pharmaceutical composition for the prevention or treatment of osteoporosis containing, as an active ingredient, a compound represented by the general formula:

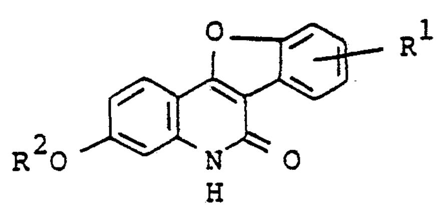

wherein $R^1$ represents a hydrogen atom, a hydroxy group or an unsubstituted or substituted straight- or branched-chain alkoxy group having 1 to 10 carbon atoms wherein the substituent is selected from a hydroxy group, a carboxy group, a carbamoyl group and an alkoxycarbonyl group having 2 to 7 carbon atoms; $R^2$ represents a hydrogen atom or an unsubstituted or substituted straight- or branched-chain alkyl group having 1 to 10 carbon atoms wherein the substituent is selected from a hydroxy group, a carboxy group, a carbamoyl group and an alkoxycarbonyl group having 2 to 7 carbon atoms, and a pharmaceutically acceptable salt thereof.

2. A pharmaceutical composition as claimed in claim 1 wherein said active ingredient is represented by the general formula:

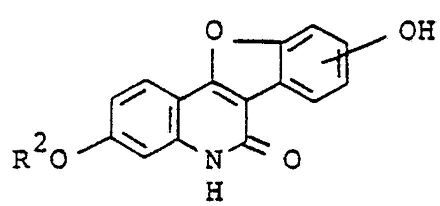

wherein $R^2$ has the same meaning as described in claim 1.

3. A pharmaceutical composition as claimed in claim 2 wherein said active ingredient is represented by the formula:

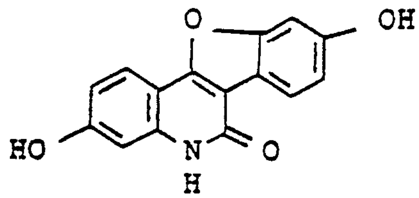

4. A pharmaceutical composition as claimed in claim 1 wherein said active ingredient is represented by the formula:

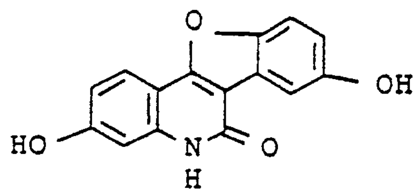

5. A pharmaceutical composition as claimed in claim 2 wherein said active ingredient is represented by the formula:

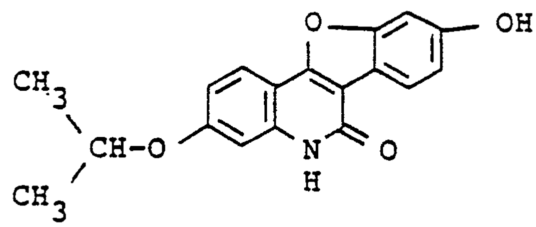

6. A pharmaceutical composition as claimed in claim 2 wherein said active ingredient is represented by the formula:

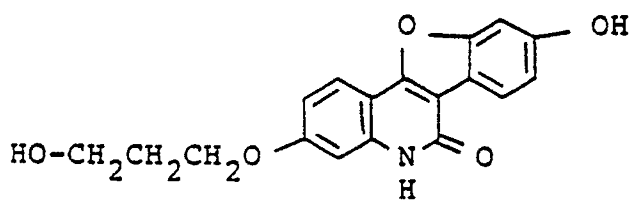

7. A method for the prevention or treatment of osteoporosis which comprises administering a therapeutically effective amount of a compound of claim 1.

8. A method for the prevention or treatment of osteoporosis which comprises administering a therapeutically effective amount of a compound of claim 2.

9. The method as claimed in claim 8 wherein said compound is represented by the formula:

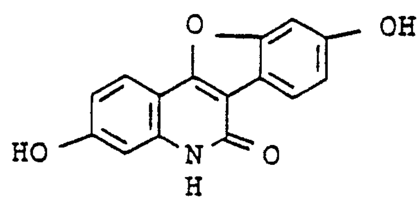

10. The method as claimed in claim 8 wherein said compound is represented by the formula:

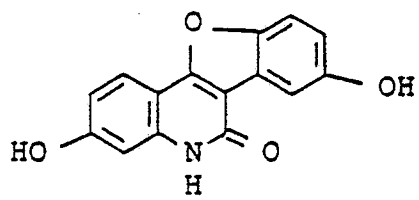

11. The method as claimed in claim 8 wherein said compound is represented by the formula:

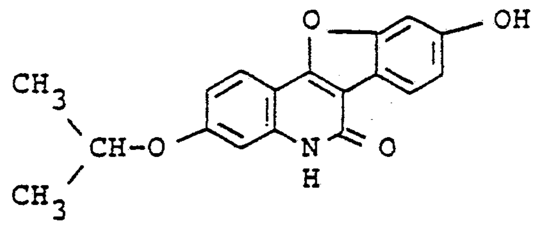

12. The method as claimed in claim 8 wherein said compound is represented by the formula:

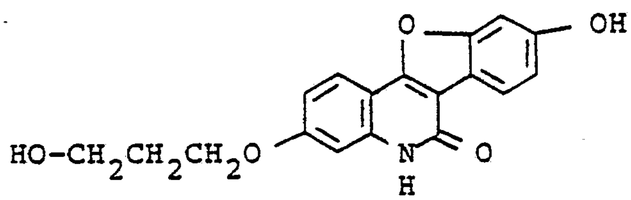

13. A compound represented by the general formula:

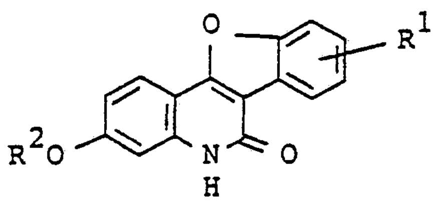

wherein $R^1$ represents a hydrogen atom, a hydroxy group or an unsubstituted or substituted straight- or branched-chain alkoxy group having 1 to 10 carbon atoms wherein the substituent is selected from a hydroxy group, a carboxy group, a carbamoyl group and an alkoxycarbonyl group having 2 to 7 carbon atoms; $R^2$ represents a hydrogen atom or an unsubstituted or substituted straight- or branched-chain alkyl group having 1 to 10 carbon atoms wherein the substituent is selected from a hydroxy group, a carboxy group, a carbamoyl group and an alkoxycarbonyl group having 2 to 7 carbon atoms, with the proviso that, when $R^1$ represents a hydroxy group, $R^2$ can not be a hydrogen atom, and with the further proviso that, when $R^1$ represents a methoxy group, $R^2$ can not be a methyl group, and a pharmaceutically acceptable salt thereof.

14. A compound as claimed in claim 13, represented by the general formula:

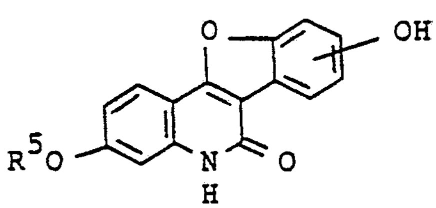

wherein $R^5$ represents an unsubstituted or substituted straight- or branched-chain alkyl group having 1 to 10 carbon atoms wherein the substituent is selected from a hydroxy group, a carboxy group, a carbamoyl group and an alkoxycarbonyl group having 2 to 7 carbon atoms.

15. The compound as claimed in claim 14, represented by the formula:

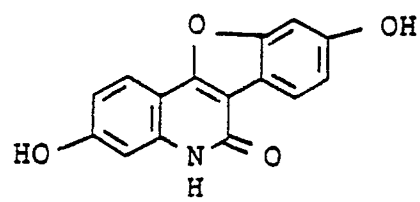

16. The compound as claimed in claim 14, represented by the formula:

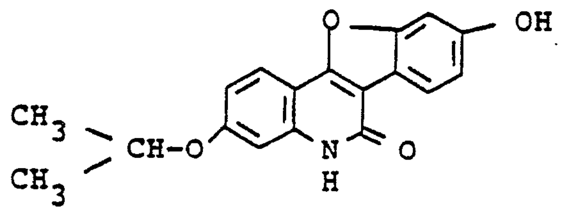

17. The compound as claimed in claim 14, represented by the formula:

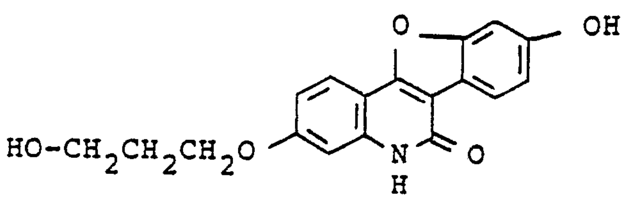

European Patent Office

# PARTIAL EUROPEAN SEARCH REPORT

which under Rule 45 of the European Patent Convention shall be considered, for the purposes of subsequent proceedings, as the European search report

Application number: EP 88304631.0

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| | JOURNAL OF HETEROCYCLIC CHEMISTRY, vol. 21, 1984<br>SEIJI YAMAGUCHI et al. "The Synthesis of Benzofuroquinolines. III. Two Dihydroxybenzofuroquinolinones" pages 737-739 | | A 61 K 31/435<br>A 61 K 31/47<br>C 07 D 491/048 |
| D,Y | * Page 737, left column, lines 1-12; page 738, formulas V,VII* | 1-4 | |
| D,X | * Page 738, formula V *<br>-- | 15 | |
| Y | EP - A2 - 0 135 172 (TAKEDA CHEMICAL INDUSTRIES, LTD.)<br>* Abstract; claims 2-4; page 1, lines 1-26 *<br>---- | 1-4 | |

TECHNICAL FIELDS SEARCHED (Int. Cl.4)

A 61 K 31/00

## INCOMPLETE SEARCH

The Search Division considers that the present European patent application does not comply with the provisions of the European Patent Convention to such an extent that it is not possible to carry out a meaningful search into the state of the art on the basis of some of the claims.

Claims searched completely: 1-6,13-17

Claims searched incompletely: -

Claims not searched: 7-12

Reason for the limitation of the search:

(method for treatment of the human or animal body by therapy - see EPC Article 52 (4))

| Place of search | Date of completion of the search | Examiner |
|---|---|---|
| VIENNA | 30-08-1988 | MAZZUCCO |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1505.1 03.82